# EUROPÄISCHE PATENTSCHRIFT

(11) **EP 1 552 958 B1**
(45) Veröffentlichungstag und Bekanntmachung des Hinweises auf die Patenterteilung: **13.07.2011**
(21) Anmeldenummer: 04003116.3
(22) Anmeldetag: 12.02.2004
(51) Int. Cl.: A61K 8/73, A61K 8/60, C09D 13/00

(54) **Mine für Schreib-, Mal- und Kosmetikzwecke**
Pencil lead for writing, painting and cosmetic purposes
Mine de crayon pour l'écriture, la peinture et la cosmétique

(30) Priorität: 09.09.2003 DE 20313963 U; 23.09.2003 DE 20314685 U
(43) Veröffentlichungstag der Anmeldung: 13.07.2005
(73) Patentinhaber: Faber-Castell AG, 90546 Stein (DE)
(72) Erfinder: von Godin, Harald, 90522 Oberasbach (DE); Appel, Reiner, 90522 Oberasbach (DE); Lugert, Gerhard, Dr., 90431 Nürnberg (DE)
(74) Vertreter: Mörtel & Höfner

(56) Entgegenhaltungen:
- EP-A- 0 570 810
- EP-A- 0 849 339
- PATENT ABSTRACTS OF JAPAN Bd. 004, Nr. 174 (C-033), 2. Dezember 1980 (1980-12-02) & JP 55 115479 A (PENTEL KK), 5. September 1980 (1980-09-05)

## Beschreibung

Die Erfindung betrifft eine Mine oder Kreide für Schreib-, Mal und Kosmetikzwecke. Bindemittelsysteme herkömmlicher Minen enthalten Cellulosederivate wie Natriumcarboxymethylcellulose (NaCMC), Hydroxyethylcellulose (HEC), Methylhydroxyethylcellulose (MHEC), Methylhydroxypropylcellulose (MHPC) oder Natriumcarboxymethylstärke (CMS). Die Wirkungsweise solche Bindemittel beruht auf ihrer Fähigkeit, in Wasser aufzuquellen. Herkömmliche Ausgangsmassen für Minen werden daher mit relativ großen Mengen an Wasser versetzt. Der Wasserzusatz bringt jedoch Nachteile mit sich: Eine wasserhaltige, etwa Füllstoffe, Farbpigmente und Additive enthaltende Zubereitung kann aufgrund mikrobiologischer Restriktionen nur mehrere Stunden oder wenige Tage aufbewahrt werden, insbesondere wenn damit Kosmetikminen hergestellt werden sollen. Nachteilig ist weiterhin, dass zum Ausformen der Minen ein konstanter Feuchtigkeitsgehalt innerhalb der Masse eingehalten werden muss, um konstante Durchmesser und gleichmäßige Minenoberflächen zu gewährleisten. Ein weiterer Nachteil ist die obligatorische Trocknung solcher Minen bei etwa 40 - 140°C. Dies erfordert nicht nur einen erhöhten Energieaufwand, sondern bedarf auch einer aufwändigen Verfahrensführung. Zum einen muss ein mit dem Wasserentzug einhergehender Schwund berücksichtigt werden. Zum anderen müssen die im feuchten Zustand flexiblen Minen zur Erhaltung der "Geradheit" in Büchsen unter Drehbewegungen bei erhöhter Temperatur und damit unter Energieaufwand getrocknet werden. Bei sog. Post-Wax-Minen werden die Minen nach dem Trocknen in einem zusätzlichen Arbeitsschritt in aufgeschmolzene Wachse oder Öle getaucht. EP-A-849 339 offenbart eine durch Extrusion hergestellte Mine, enthaltend ein Polyol als Bindemittel.

JP-A-55 115 479 offenbart Farbminen, hergestellt durch Extrusion, enthaltend Hexitol.

Aufgabe der Erfindung ist es, eine Mine für Schreib- Mal- und Kosmetikzwecke anzugeben, mit der die geschilderten Nachteile umgangen sind.

Diese Aufgabe wird mit einer Mine nach Anspruch 1 gelöst.

Danach ist die Mine durch thermoplastische Verarbeitung, etwa im Spritzgussverfahren oder vorzugsweise durch Extrusion einer wenigstens einen Zucker und/oder Zuckeralkohol in fester Form enthaltenden Minen-Ausgangsmasse bei erhöhter, ein Schmelzen des Zuckers und/oder Zuckeralkohols bewirkenden Temperatur hergestellt. Ein Erweichen bzw. Schmelzen des Zuckers oder gegebenenfalls auch anderer Bestandteile lässt sich naturgemäß in wässriger Lösung nicht erreichen. Die Minen-Ausgangsmasse ist daher wasserfrei oder enthält höchstens 2 % Wasser. Der Erfindung liegt dabei die Idee zugrunde, als Bindemittel Stoffe, insbesondere naturnahe, zu verwenden, die bei der Ausformung der Mine, etwa durch Extrusion der Minenmasse aufschmelzen und die übrigen Minenbestandteile binden. Dabei liegt nach dem Erkalten der Minenmasse bereits die fertige Mine vor, so dass ein aufwändiges Entfernen von Wasser oder sonstigen Lösungsmitteln entfällt. Die Minen-Ausgangsmischung und auch die Mine können bei Bedarf über mehrere Wochen oder Monate gelagert werden, wobei wegen des fehlenden Wassergehalts eine mikrobiologische Beeinträchtigung kaum zu befürchten ist.

Bei einer besonders bevorzugten Variante setzt sich das Bindemittelsystem der Mine aus wenigstens einem Zucker und/oder Zuckeralkohol und einem Cellulose- und/oder Stärkederivat zusammen. Es hat sich überraschenderweise herausgestellt, dass auch eine solche Mischung thermoplastisch erweichbar und im Extrusionsverfahren verarbeitbar ist. Weiterhin zeigte es sich, dass sich mit einem derartigen Bindemittelsystem Minen herstellen lassen, die gegenüber ausschließlich Zucker und/oder Zuckeralkohole als thermoplastisch erweichbares Bindemittel enthaltenden Minen eine erhöhte Bruchfestigkeit aufweisen. Die thermoplastisch erfindungsgemäß hergestellten Minen zeichnen sich darüber hinaus durch eine sehr gleichmäßige, homogene Abgabe auf Papier aus. Im Vergleich zu herkömmlichen Minen tritt ein Stauben oder Bröseln der Minenmasse während der Applikation auf Papier kaum auf.

Ein Verschmelzen von Zuckern mit Cellulosen und Stärken bzw. deren Derivaten gelingt oft nur unter Anwendung eines erhöhten Drucks, da Zucker wie Glucose, Fructose, Saccharose, Mannose u.a. als auch Cellulosederivate und Stärkederivate zwischen 100 und 220°C Zersetzungserscheinungen aufweisen. Leichter gelingt die homogene Verschmelzung bzw. Vermischung bei Verwendung von Zuckeralkoholen z. B. Sorbit, Mannit, Xylit, Adonit, Arabit, Dulcit, Threit, oder von Pentaerythrit. Diese Substanzen lassen sich oberhalb von 100°C ohne Anwendung erhöhten Drucks zunächst wasserklar aufschmelzen und nehmen Cellulosen oder Stärken bzw. deren Derivate überraschenderweise problemlos auf. Als Cellulosederivate besonders geeignet sind Natriumcarboxymethylcellulose (NaCMC) oder auch Natriumcarboxy-methylstärke (CMS). Hier gelingt eine innige Verbindung in Schnellmischern oder Extrudern bei Temperaturen von 80 - 120°C. Eine innige Vermischung bzw. Verbindung kann auch dadurch gefördert werden dass während des Vermischungs- bzw. Compoundierungsprozesses Cellulosen und/oder Stärken und Zucker/Zuckeralkohole mit 5 % - 30 % Wasser versetzt werden, wobei während der Vermischung der Ausgangsstoffe, etwa bei der Compoundherstellung im Extruder, das vorhandene Wasser bis auf einen Restgehalt von kleiner gleich 2 % verdunstet wird, so dass dann ein Aufschmelzen des Zuckers oder Zuckeralkohols möglich ist und Minen mit einem entsprechend geringen Feuchtigkeitsgehalt extrudiert werden können.

Eine Masse, die sich besonders zur Minenherstellung eignet, enthält ein Bindemittelsystem, das aus 0,8 % bis 18 %, vorzugsweise 0,8 % bis 8 % Cellulosederivat und/oder Stärkederivat und 4 % bis 40 %, vorzugsweise 4 % bis 30 % Zucker und/oder Zuckeralkohol gebildet ist.

Das Eigenschaftsspektrum der Mine, etwa deren Abstrichverhalten, wird durch Zugabe von Füllstoffen mit einem Anteil von 40 % bis 70 % und fett- oder wachsartiger Stoffe mit einem Anteil von 5 % bis 25 % beeinflusst. Es werden Minen erhalten, die etwa den bekannten Post-Wax-Minen entsprechen, wobei jedoch ein zusätzlicher Arbeitsschriftt (Eintauchen der Mine in geschmolzenes Wachs oder Öl) entfällt. Als Füllstoffe eignen sich vor allem Kaolin, Talkum, Glimmer und Titandioxid.

Durch Verwendung fett- oder wachsartiger Stoffe, die sowohl lipophile als auch hydrophile Eigenschaften besitzen, etwa das Na-Salz einer Fettsäure, z.B. Natriumstearat, lassen sich wasserlösliche bzw. wasserquellbare Minen herstellen.

Für Farbminen oder Kreiden für den Schreib- und Kosmetikbereich können folgende Basisformulierungen angegeben werden:

Minen mit einem Zuckeralkohol als alleiniges thermoplastisch erweichbares Bindemittel:

| | |
|---|---|
| Zucker und/oder Zuckeralkohole | 4 bis 30 % |
| Fett- oder wachsartige Stoffe | 5 bis 25 % |
| Füllstoffe | 40 bis 70 % |

Rest: je nach Bedarf z.B. Additive wie Aromen, Gleitmittel, Farbmittel, Wachse, Fette und Emulgatoren.

Mine mit einer Mischung aus Cellulose - / Stärkederivaten und Zucker / Zuckeralkoholen:

| | |
|---|---|
| Cellulosederivate und/oder Stärkederivate | 0,5 bis 18 % |
| Zucker und/oder Zuckeralkohole | 4 bis 40 % |
| Füllstoffe | 40 bis 70 % |

Rest: je nach Bedarf z.B. Additive wie Aromen, Gleitmittel, Farbmittel, Wachse, Fette und Emulgatoren.

### Beispiel 1:

Aquarellierbare, grüne Farbminen, Durchmesser 3,0 mm.

| | |
|---|---|
| Mannit | 6,0 % |
| Sorbit | 3,0 % |
| NaCMC (Walocel CRT 100, Wolff, Walsrode) | 2,0 % |
| Natriumstearat | 10,0 % |
| Ethoxy (80) Cetostearyl Alcohol (= Emulgator, CAS 68439-49-6) | 8,0 % |
| Kaolin | 64,0 % |
| Pigment Green 7 (C.I.74260) | 7,0 % |

Die Rohmaterialien werden in einem Schnellmischer zwischen 80 und 100°C homogen zu einem Compound vermischt. Neben einer trockenen Homogenisierung kann zur Beschleunigung des Prozesses zunächst ein Wasseranteil von 15 bis 20 % zugegeben werden und während des Mischvorgangs im Schnellmischer bis auf eine Restfeuchtigkeit von max. 2 % wieder verdunstet werden. Das erhaltene feine Granulat ist über mehrere Wochen hinweg lagerbar und kann bei Bedarf auf einen Schneckenextruder (bevorzugt 2-Schneckenextruder) bei ca. 150°C zu Minen ausgeformt werden. Die erhaltenen thermoplastisch ausgeformten Minen zeigen aufgrund des vorhandenen Emulgator + Natriumstearatzusatzes eine gute Aquarellierbarkeit.

### Beispiel 2

Eine Vergleichsmine, die ohne dem Zusatz von Natriumcarboxycellulose auf die oben beschriebene Weise hergestellt wurde:

| | |
|---|---|
| Mannit | 6,0 % |
| Sorbit | 3,0 % |
| Natriumstearat | 10,0 % |
| Ethoxy (80) Cetostearyl Alcohol (CAS 68439-49-6) | 8,0 % |
| Kaolin | 66,0 % |
| Pigment Green 7 (C.I. 74260) | 7,0 % |

Die Masse lässt sich ebenfalls zu Minen extrudieren, weist aber eine Querbruchfestigkeit auf, die mit ca. 25 % unter der Querbruchfestigkeit der Minen gem. Beispiel 1 liegt. Daraus zeigt sich, dass sich die höhere Festigkeit der Minen nach Beispiel 1 aus dem Zusammenspiel zwischen Zucker, insbesondere Zuckeralkoholen mit Celluloseethern z.B. Natriumcarboxymethylcellulose ergibt.

### Beispiel 3:

Beschreibt eine nicht aquarellierbare runde Kreide, Durchmesser 10 mm.

| | |
|---|---|
| Mannit | 12,0 % |
| Pentaerythrit | 4,0 % |
| Natriumcarboxymethylstärke (CMS) | 5,0 % |
| Talkum | 66,6 % |
| Fettalkohol + ca. 50 Mol/kg Ethylenoxid (Genapol T 500 Pulver, | 6,0 % |
| Hersteller: Clariant) Pigment Yellow 3 (C.I.11710) | 6,4 % |

Die Extrusion der gelben.Kreiden erfolgt. Nach einer Compound-Herstellung im Schnellmischer bei ca. 90°C erfolgt eine Extrusion der Kreiden bei einer Temperatur zwischen 140°C und 160°C auf einem 2-Schnecken-Extruder.

### Beispiel 4:

Wasserfeste blaue Mine, Durchmesser 4,0 mm

| | |
|---|---|
| Mannit | 9,0 % |
| Xylit | 4,0 % |
| Bindemittelmischung bestehend aus CMC und CMS (= Crayon B 300 P, Lieferant: Albon-Chemie) | 3,0 % |
| Natriumstearat | 5,0 % |
| Kaolin | 67,0 % |
| Stearinsäure | 2,9 % |
| Paraffinwachs | 5,1 % |
| Pigment Blue 15 : 1 (C.I. 74160) | 4,0 % |

Nach Compound-Herstellung auf einem 2-Schnecken-Extruder mit einer Temperaturführung zwischen 85 und 100°C erfolgt Minenextrusion bei 130 - 150°C. Die weitgehende Wasserfestigkeit ist durch den Wachs- und Fettsäurezusatz bedingt.

Alle Prozentangaben sind Gewichtsprozent.

### Hersteller-Adressen:

Clariant GmbH, D-65849 Sulzbach
Albon Chemie, D-72555 Metzingen
Crode GmbH, D 41334 Nettetal
Wolff Walsrode GmbH, D-29655 Walsrode

## Patentansprüche

1. Mine für Schreib- Mal- und Kosmetikzwecke,
**dadurch gekennzeichnet,**
**dass** sie durch thermoplastische Verarbeitung einer wenigstens einen Zucker und/oder Zuckeralkohol oder Pentaerythrit, wenigstens ein Cellulose- und/oder Stärkederivat und höchstens 2 % Wasser enthaltenden Minen-Ausgangsmasse bei erhöhter, ein Erweichen des Zuckers und/oder Zuckeralkohols bewirkenden Temperatur hergestellt ist.

2. Mine nach Anspruch 1,
**dadurch gekennzeichnet,**
**dass** sie wenigstens einen Zuckeralkohol aus der Gruppe Mannit, Sorbit, Xylit, Adonit, Arabit, Dulcit, und Threit enthält.

3. Mine nach Anspruch 1 oder 2,
**dadurch gekennzeichnet,**
**dass** sie als Zucker Xylose enthält.

4. Mine nach Anspruch 3,
**dadurch gekennzeichnet,**
**dass** sie Natriumcarboxymethylcellulose und/oder Natriumcarboxy-methylstärke enthält.

5. Mine nach Anspruch 3 oder 4,
**dadurch gekennzeichnet,**
**dass** sie 0,5 % bis 18 % Cellulosederivat und/oder Stärkederivat sowie 4 % bis 40 % Zucker und/oder Zuckeralkohol enthält.

6. Mine nach Anspruch 5,
**dadurch gekennzeichnet,**
**dass** sie 0,5 bis 8 % Cellulosederivat und/oder Stärkederivat sowie 4 bis 30 % Zucker und/oder Zuckeralkohol enthält.

7. Mine nach einem der Ansprüche 1 bis 6, -
**dadurch gekennzeichnet,**
**dass** sie wenigstens einen Füllstoff mit einem Anteil von 40 bis 70 % enthält.

8. Mine nach Anspruch 7,
**dadurch gekennzeichnet,**
**dass** sie wenigstens einen Füllstoff aus der Gruppe Talkum, Kaolin, Glimmer und Titandioxid enthält.

9. Mine nach einem der Ansprüche 1 bis 8,
**dadurch gekennzeichnet,**
**dass** sie wenigstens einen fett- oder wachsartigen Stoff enthält.

10. Mine nach Anspruch 9,
**dadurch gekennzeichnet,**
**dass** sie als fett- oder wachsartigen Stoff eine Fettsäure und/oder ein Fettsäurederivat enthält.

11. Mine nach Anspruch 9 oder 10,
**dadurch gekennzeichnet,**
**dass** sie 5 % bis 25 % des fett- oder wachsartigen Stoffs enthält.

## Claims

1. Lead for writing, painting and cosmetic purposes,
**characterized in that**
it is produced by thermoplastic processing of a lead starting mass comprising at least one sugar and/or sugar alcohol or pentaerythritol, at least one cellulose and/or starch derivative and at most 2% water at an elevated temperature which brings about softening of the sugar and/or sugar alcohol.

2. Lead according to Claim 1,
**characterized in that**
it comprises at least one sugar alcohol from the group consisting of mannitol, sorbitol, xylitol, adonitol, arabitol, dulcitol and threitol.

3. Lead according to Claim 1 or 2,
**characterized in that**
it comprises xylose as sugar.

4. Lead according to Claim 3,
**characterized in that**
it comprises sodium carboxymethylcellulose and/or sodium carboxymethylstarch.

5. Lead according to Claim 3 or 4,
**characterized in that**
it comprises 0.5% to 18% cellulose derivative and/or starch derivative, and 4% to 40% sugar and/or sugar alcohol.

6. Lead according to Claim 5,
**characterized in that**
it comprises 0.5 to 8% cellulose derivative and/or starch derivative, and 4 to 30% sugar and/or sugar alcohol.

7. Lead according to any of Claims 1 to 6,
**characterized in that**
it comprises at least one filler in an amount of from 40 to 70%.

8. Lead according to Claim 7,
**characterized in that**
it comprises at least one filler from the group consisting of talc, kaolin, mica and titanium dioxide.

9. Lead according to any of Claims 1 to 8,
**characterized in that**
it comprises at least one fat-like or wax-like substance.

10. Lead according to Claim 9,
**characterized in that**
it comprises a fatty acid and/or a fatty acid derivative as fat-like or wax-like substance.

11. Lead according to Claim 9 or 10,
**characterized in that**
it comprises 5% to 25% of the fat-like or wax-like substance.

## Revendications

1. Mine à des fins d'écriture, de peinture et de cosmétique,
**caractérisée**
**en ce qu'**elle est fabriquée par un traitement thermoplastique d'au moins un sucre et/ou d'un alcoolsucre ou de pentaérythrite, d'au moins un dérivé de cellulose et/ou d'amidon et d'une masse de départ de mine contenant au plus 2% d'eau, à une température élevée provoquant un ramollissement du sucre et/ou de l'alcoolsucre.

2. Mine suivant la revendication 1,
**caractérisée**
**en ce qu'**elle contient au moins un alcoolsucre dans le groupe du mannite, du sorbite, du xylite, de l'adonite, de l'arabite, du dulcite et du thréite.

3. Mine suivant la revendication 1 ou 2,
**caractérisée**
**en ce qu'**elle contient du xylose comme sucre.

4. Mine suivant la revendication 3,
**caractérisée**
**en ce qu'**elle contient de la carboxyméthylcellulose sodique et/ou du carboxy-méthylamidon sodique.

5. Mine suivant la revendication 3 ou 4,
**caractérisée**
**en ce qu'**elle contient de 0,5% à 18% de dérivé de cellulose et/ou de dérivé d'amidon, ainsi que de 4% à 40% de sucre et/ou d'alcoolsucre.

6. Mine suivant la revendication 5,
**caractérisée en ce qu'**elle contient de 0,5 à 8 % de dérivé de cellulose et/ou de dérivé d'amidon, ainsi que de 4 à 30% de sucre et/ou d'alcoolsucre.

7. Mine suivant la revendication 1 à 6,
**caractérisée en ce qu'**elle contient au moins une charge en une proportion de 40 à 70%.

8. Mine suivant la revendication 7,
**caractérisée en ce qu'**elle contient au moins une charge dans le groupe du talc, du kaolin, du mica et du dioxyde de titane.

9. Mine suivant l'une des revendications 1 à 8,
**caractérisée en ce qu'**elle comprend au moins une substance de type graisse ou cire.

10. Mine suivant la revendication 9,
**caractérisée en ce qu'**elle contient, comme substance du type graisse ou cire, un acide gras et/ou un dérivé d'acide gras.

11. Mine suivant la revendication 9 ou 10,
**caractérisée en ce qu'**elle contient de 5 % à 25 % de la substance du type graisse ou cire.
